# EUROPÄISCHE PATENTSCHRIFT

(11) **EP 1 070 490 B1**
(45) Veröffentlichungstag und Bekanntmachung des Hinweises auf die Patenterteilung: **01.10.2003**
(21) Anmeldenummer: 99114202.7
(22) Anmeldetag: 23.07.1999
(51) Int. Cl.: A61F 2/36

(54) **Hüftprothesenschaft**
Stem for a hip prosthesis
Tige d'une prothèse de hanche

(43) Veröffentlichungstag der Anmeldung: 24.01.2001
(73) Patentinhaber: Aesculap AG & Co. KG, 78532 Tuttlingen (DE)
(72) Erfinder: Bataille, Dr. Jean-Francois Clinique Dr.Montagard, 84000 Avignon (FR); Panisset,Dr.Jean-Claude Clinique des Cedres, 38100 Grenoble (FR); Collin, Dr. Michel Clinigue Chirurgicale Labat, 64300 Orthez (FR); Daubresse, Dr. Francois, 5170 Profonde Ville (BE); Mainard, Professor Didier, 54000 Nancy (FR); Rifai, Dr. Yassar Hopital Maison Blanche, 51092 Reims Cedex (FR); Reinbold, Dipl.-Ing. Ulrich, 78532 Tuttlingen (DE)
(74) Vertreter: Hoeger, Stellrecht & Partner

(56) Entgegenhaltungen:
- DE-A- 2 627 569
- FR-A- 2 676 359
- FR-A- 2 756 727
- US-A- 5 156 627

## Beschreibung

Die Erfindung betrifft einen Hüftprothesenschaft. Derartige Hüftprothesenschäfte werden vielfältig eingesetzt, um mit Hilfe von Knochenzement oder zementfrei im Inneren des entsprechend vorbereiteten Oberschenkelknochens verankert zu werden. Dabei ist es für die Vorbereitung wichtig, den Markraum des Oberschenkelknochens möglichst optimal an die Form des Hüftprothesenschaftes anzupassen, dies erfolgt bei Hüftprothesenschäften, die einzementiert werden sollen, üblicherweise mit einem Hüftprothesenschaft, der von der Innenwand des vorbereiteten Markraumes einen geringen Abstand zur Aufnahme des Knochenzementes aufweist, während bei der zementfreien Verankerung die Innenwand des vorbereiteten Markraumes mit den Außenkonturen des Hüftprothesenschaftes möglichst gut zusammenpassen sollte.

Ein wie im Oberbegriff von Anspruch 1 beschriebener Hüftprothesenschaft ist aus DE 26 27 569 A1 bekannt.

Bisher ist es üblich gewesen, den Markraum im Oberschenkelknochen manuell oder mittels eines handgeführten Ausräuminstrumentes vorzubereiten, dabei sind jedoch nicht die gewünschten Genauigkeiten erzielbar. Es ist wünschenswert, die Vorbereitung des Markraumes durch ein gesteuertes Werkzeug, also durch einen Ausräumroboter, vornehmen zu lassen. Dabei ist es allerdings schwierig, den Markraum in beliebiger Weise zu formen, die verwendeten gesteuerten Ausräumwerkzeuge machen es vielmehr in Einzelfällen notwendig, die erzielbaren Querschnittsformen und Verläufe des Markraumes auf bestimmte Geometrien einzuschränken.

Es ist Aufgabe der Erfindung, einen gattungsgemäßen Hüftprothesenschaft so auszubilden, daß er optimal in einen Markraum eingepaßt werden kann, der in dieser Weise durch ein gesteuertes Werkzeug oder einen Ausräumroboter vorbereitet worden ist.

Diese Aufgabe wird erfindungsgemäß gelöst durch einen Hüftprothesenschaft, der die Merkmale des Patentanspruches 1 aufweist.

Es hat sich herausgestellt, daß eine solche Formgebung des Stiels optimal geeignet ist zum Einpassen in Markräume, die durch einen Ausräumroboter vorbereitet worden sind. Insbesondere die relativ großen Radien zwischen Seitenflächen einerseits und der vorderen und hinteren Außenfläche andererseits tragen dabei dazu bei, daß eine sehr gleichmäßige Anpassung der Außenkontur des Stieles an die Innenkontur des vorbereiteten Markraumes möglich wird, so daß bei der zementfreien Verankerung eine gute flächige Anlage des Stieles an der Innenwand des Markraumes erzielt werden kann, bei der Zementverankerung andererseits ein Zwischenraum zwischen Stiel und Markrauminnenwand, der im wesentlichen überall die gleiche Dicke aufweist. Da der Radius der abgerundeten Kanten maximal 5 mm beträgt, erfolgt keine vollständige Ausrundung, sondern immer noch ein Übergehen von einer ebenen Seitenfläche in die vordere und hintere Außenfläche. Diese kann einen ebenen Mittelabschnitt aufweisen, im Extremfall ist es aber auch möglich, daß die abgerundeten Kanten unmittelbar zusammentreffen und somit eine vollständig gerundete Außenfläche an der Vorderseite und an der Rückseite des Stieles ausbilden.

Bei einer bevorzugten Ausführungsform ist vorgesehen, daß die Stege im Querschnitt je einen nach außen gebogenen Abschnitt und einen daran stetig anschließenden, nach innen gebogenen, stetig in die Seitenflächen des Kopfteils übergehenden Abschnitt aufweisen, wobei der Radius des nach außen gebogenen Abschnittes mindestens 2,9 mm beträgt. Diese Stege oder Rippen auf den Seitenflächen dienen der Verankerung des Hüftprothesenschaftes im Markraum, und die beschriebene Struktur führt dazu, daß diese Stege eine gleichmäßige, kantenfreie Struktur aufweisen, die optimal an die Markrauminnenwand eines Markraums anpaßbar ist, der durch ein gesteuertes Werkzeug oder einen Roboter vorbereitet worden ist.

Dabei ist es vorteilhaft, wenn die nach außen gebogenen Abschnitte der Stege stetig ineinander übergehen, insbesondere kann dabei der Radius der nach außen gebogenen Abschnitte von den Seitenflächen des Kopfteils bis zu ihrem Zusammentreffen an der hinteren Außenfläche zunehmen.

Der Radius des nach innen gebogenen Abschnitts liegt dabei in der Größenordnung zwischen 3 und 4 mm, es handelt sich also auch um einen relativ großen Radius, der scharfe Kanten vermeidet.

Die Höhe der Stege relativ zu den Seitenflächen des Kopfteils kann zwischen 2 und 3 mm liegen.

Es ist dabei günstig, wenn die Stege vom unteren Ende des Kopfteils bis an dessen Oberkante verlaufen und von ihrem unteren Ende, welches parallel zur Längsachse des Stiels verläuft, bis zu ihrem oberen Ende in Richtung auf den Steckkonus gekrümmt sind.

Vorzugsweise kann der Krümmungsradius der Stege zwischen 100 und 140 mm liegen, günstig ist insbesondere ein Krümmungsradius in der Größenordnung von 120 mm.

Die Seitenflächen des Stieles können völlig parallel zueinander verlaufen, es ist aber besonders vorteilhaft, wenn diese ebenen Seitenflächen vom distalen Ende des Hüftprothesenschaftes ausgehend um einen Winkel zwischen 1,5° und 2,5° divergieren. Dies erleichtert sowohl das Einsetzen als gegebenenfalls auch das Herausziehen eines implantierten Prothesenschafts.

Ebenso können die vordere und die hintere Außenfläche des Stiels vollständig parallel zueinander ausgebildet sein, es ist aber vorteilhaft, wenn die vordere und die hintere Außenfläche des Stiels von dessen distalem Ende ausgehend um einen Winkel zwischen 2,5° und 3,5° divergieren.

Die Erfindung bezieht sich auch auf einen Satz von Hüftprothesen mit den vorstehend beschriebenen Merkmalen, wobei zusätzlich vorgesehen ist, daß der Abstand der vorderen und hinteren Außenflächen des Stieles bei den verschiedenen Hüftprothesenschäften dieses Satzes zwischen 8 und 17 mm abgestuft ist und daß die Radien der abgerundeten Kanten der Seitenflächen des Stieles und die Radien der nach außen gebogenen Abschnitte der Stege von einem Mindestradius von 2,9 mm bis zu einem Maximalradius von 4,7 mm proportional zum Abstand der vorderen und hinteren Außenflächen des Stieles zunehmen.

In diesem Satz von Hüftprothesenschäften sind also unterschiedliche Größen der Hüftprothesenschäfte vereinigt, wobei bei unterschiedlichen Größen der Abstand der vorderen und hinteren Außenflächen des Stieles zwischen 8 und 17 mm stufenweise zunimmt. Entsprechend dieser Zunahme ändern sich auch die Radien der abgerundeten Kanten der Seitenflächen des Stieles und der nach außen gebogenen Abschnitte der Stege, und zwar proportional zur Zunahme dieses Abstandes der vorderen und hinteren Außenflächen des Stieles, so daß bei einem Hüftprothesenschaft mit einem Abstand von 8 mm zwischen vorderer und hinterer Außenfläche des Stiels der genannte Radius etwa 2,9 mm beträgt, bei einem Hüftprothesenschaft mit 17 mm Abstand zwischen der vorderen und der hinteren Außenfläche des Stieles dagegen etwa 4,7 mm. Man erhält dadurch ein sehr ausgewogenes Verhältnis zwischen den Abmessungen des Stieles und den Abrundungen, so daß bei unterschiedlichen Teilen des Satzes in allen Fällen eine optimale Anpassung an einen Markraum möglich ist, der mittels eines gesteuerten Werkzeuges oder eines Roboters hergestellt worden ist.

Es kann weiterhin vorgesehen sein, daß auch der Abstand der Seitenflächen des Stieles zwischen einem Minimalmaß von 6,5 mm und einem Maximalmaß von 10,6 mm proportional zum Abstand der vorderen und hinteren Außenflächen des Stieles zunimmt.

Bei einer weiteren bevorzugten Ausführungsform ist vorgesehen, daß die Gesamtlänge des Stieles zwischen einem Minimalmaß von 140 mm und einem Maximalmaß von 183 mm proportional zum Abstand der vorderen und hinteren Außenflächen des Stieles zunimmt.

In allen Fällen sind also bestimmte Maße in diesem Satz unmittelbar abhängig von dem Abstand der vorderen und hinteren Außenflächen des Stieles und damit der Größe des Hüftprothesenschaftes, während andere Dimensionierungen für alle Teile des Satzes unverändert bleiben, beispielsweise die Höhe der Stege, die Divergenzwinkel der Seitenflächen und der Außenflächen des Stieles, der Radius des nach innen gebogenen Abschnittes der Stege oder der Krümmungsradius der Stege über die Höhe des Hüftprothesenschaftes. Es hat sich herausgestellt, daß die Teile des in dieser Weise abgestuften Satzes alle besonders geeignet sind, um in robotervorbereitete Markraumhöhlen eingesetzt zu werden.

Die nachfolgende Beschreibung einer bevorzugten Ausführungsform der Erfindung dient im Zusammenhang mit der Zeichnung der näheren Erläuterung. Es zeigen:
- Figur 1:: eine Vorderansicht eines Hüftprothesenschaftes mit Angabe verschiedener Dimensionierungsgrößen;
- Figur 2:: eine Seitenansicht des Hüftprothesenschafts der Figur 1;
- Figur 3:: eine Schnittansicht längs Linie 3-3 in Figur 4 und
- Figur 4:: eine Schnittansicht längs Linie 4-4 in Figur 1.

Der in der Zeichnung dargestellte Hüftprothesenschaft 1 weist einen Stiel 2 mit gerader Längsachse auf, der von zwei ebenen Seitenflächen 3, 4, einer hinteren Außenfläche 5 und einer vorderen Außenfläche 6 begrenzt wird. Die Seitenflächen 3 und 4 gehen über abgerundete Kanten 7 in die Außenflächen 5 und 6 über, diese Kanten 7 haben einen Krümmungsradius Ra und bilden somit einen kantenfreien, sanften Übergang zwischen den Seitenflächen und den beiden Außenflächen aus.

Der Stiel 2 mündet in ein Kopfteil 8 des Hüftprothesenschaftes 1 ein, welches ebenfalls durch zwei ebene Seitenflächen 9, 10 begrenzt wird, die stetig in die Seitenflächen 3, 4 übergehen und praktisch eine Verlängerung dieser Seitenflächen 3 und 4 bilden. Das Kopfteil 8 verbreitert sich in einer Ebene, die parallel zu den Seitenflächen 9 und 10 verläuft, von unten nach oben, wobei die vordere Außenfläche 6 des Stieles 2 in eine gekrümmte Außenfläche 11 an der Vorderseite des Hüftprothesenschaftes 1 übergeht. Am Ende der Außenfläche 11 trägt das Kopfteil 8 einen Steckkonus 12 für eine in der Zeichnung nicht dargestellte Gelenkkugel, dessen Längsachse gegenüber der Längsachse des Stieles 2 um einen Winkel geneigt ist, der in der Größenordnung von 135° liegt.

Beide Seitenflächen 9 und 10 tragen an ihrem der Außenfläche 11 gegenüberliegenden Ende je einen seitlich abstehenden, rippenförmigen Steg 14, 15, der sich vom unteren Ende des Kopfteiles 8 bis an dessen oberes Ende erstreckt. Dabei verläuft der Steg an seinem unteren Ende im wesentlichen parallel zur Längsachse des Stieles 2, er ist über seinen gesamten Verlauf bogenförmig in Richtung auf die vordere Außenfläche 11 und damit auf den Steckkonus 12 hin gekrümmt und bildet somit eine nach vorne gekrümmte Rippe aus, der Krümmungsradius Rs liegt dabei zwischen 100 und 140 mm, vorzugsweise bei 120 mm.

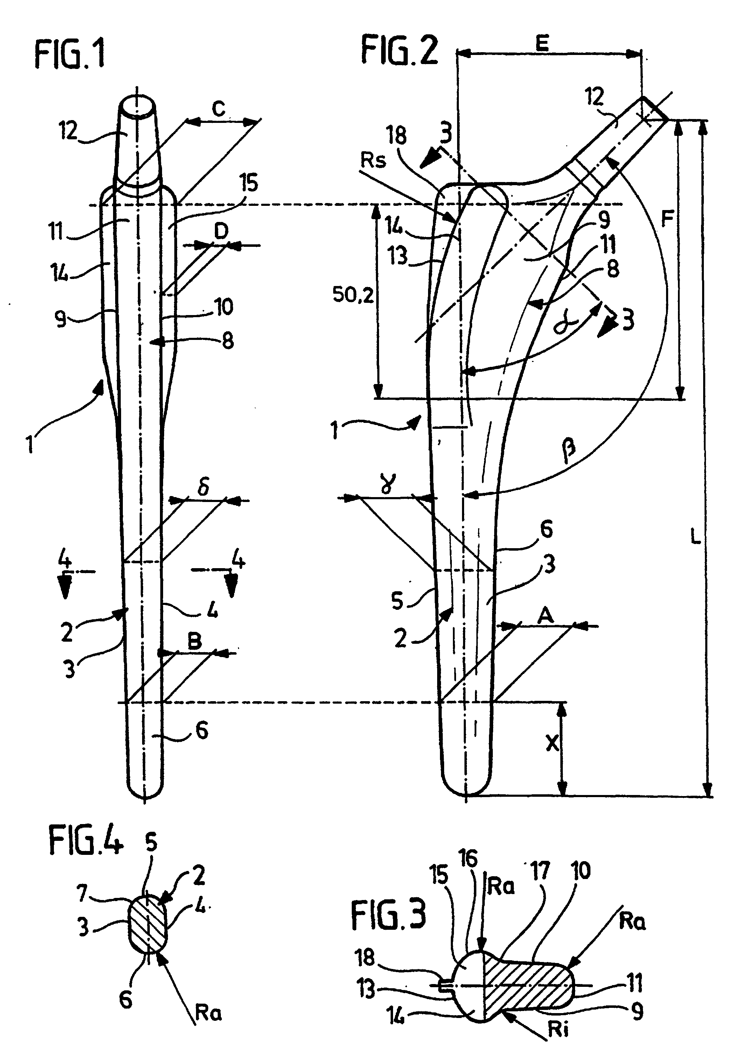

Im Querschnitt werden beide Stege zusammengesetzt durch einen nach außen gebogenen Abschnitt 16 und einen sich stetig daran anschließenden, nach innen gebogenen Abschnitt 17, der stetig in die jeweilige Seitenfläche 9 bzw. 10 übergeht (Figur 3). Der nach außen gebogene Abschnitt a weist einen kleinsten Krümmungsradius Ra auf, der an den Außenkanten der Stege 14 und 15 lokalisiert ist, von dort ausgehend nimmt der Krümmungsradius zur Rückseite des Hüftprothesenschaftes 1 ab, und die nach außen gekrümmten Abschnitte 16 gehen an der Rückseite des Hüftprothesenschaftes unter Ausbildung einer hinteren Außenfläche 13 ineinander über (Figur 3). Längs der Linie, an der diese nach außen gekrümmten Abschnitte 16 in der Längsmittelebene des Hüftprothesenschaftes 1 zusammentreffen, steht parallel zur Längsmittelebene eine relativ dünner Steg 18 ab, der nach Art einer Finne oder Rippe als Verdrehsicherung dient und die Verankerung des Hüftprothesenschaftes 1 im Markraum fördert.

Der nach innen gebogene Abschnitt 17, der den nach außen gebogenen Abschnitt 16 stufenfrei und stetig mit den Seitenflächen 9 bzw. 10 verbindet, weist einen Krümmungsradius Ri auf, der zwischen 3 und 4 mm liegt, vorzugsweise bei 3,5 mm.

Die Höhe D der Stege 14 und 15 relativ zu den Außenflächen 9 bzw. 10 liegt zwischen 2 und 3 mm, vorzugsweise bei 2,5 mm.

Die Seitenflächen 3 und 4 des Stiels 2 divergieren bei dem in der Zeichnung dargestellten Ausführungsbeispiel vom unteren, distalen Ende des Stieles 2 nach oben hin geringfügig, der öffnungswinkel δ kann zwischen 1,5 und 2,5° liegen, vorzugsweise bei 2°.

Auch die Außenflächen 5 und 6 des Stieles 2 divergieren in dieser Richtung geringfügig, der öffnungswinkel γ kann zwischen 2,5 und 3,5° liegen, vorzugsweise bei 3°.

Die Gesamtlänge L des Hüftprothesenschaftes 1 liegt zwischen etwa 140 und 185 mm, der Abstand E des auf den Steckkonus 12 aufgesetzten Gelenkkopfes von der Längsachse des Stieles 2 liegt zwischen 37 und 49 mm.

Der Abstand A der vorderen Außenfläche 6 und der hinteren Außenfläche 5 des Stieles 2 liegt in der Größenordnung von 8 bis 17 mm, wobei dieser Abstand A in einem Abstand X vom distalen Ende des Stieles 2 bestimmt wird, der je nach Größe des Hüftprothesenschaftes 1 zwischen 18 und 27 mm liegt. In derselben Höhe wird der Abstand B der beiden Seitenflächen 3 und 4 des Stieles 2 bestimmt, dieser liegt zwischen etwa 6,5 und 10,6 mm.

Die Gesamtbreite C des Kopfteiles 8 an dessen proximalem Ende beträgt von der Außenseite des Steges 14 bis zur Außenseite des Steges 15 etwa 15 bis 19,5 mm.

Die angegebenen Dimensionen können in den beschriebenen Grenzen variieren, es ist jedoch ganz besonders vorteilhaft, wenn mehrere unterschiedlich dimensionierte Hüftprothesenschäfte 1 in einem Satz zur Verfügung stehen, wobei sich diese Mitglieder des Satzes zunächst dadurch unterscheiden, daß der Abstand A der vorderen Außenfläche 6 von der hinteren Außenfläche 5 im Abstand X vom distalen Ende des Stieles 2 abgestuft ist zwischen einem Mindestmaß von 8 mm und einem Höchstmaß von 17 mm, beispielsweise können diese Abstufungen jeweils 1 mm betragen.

Entsprechend der jeweils gewählten Größe A ändern sich andere Dimensionen innerhalb des Satzes entsprechend. Es handelt sich dabei u.a. um den Abstand B der beiden Seitenflächen 3 und 4 im Abstand X vom distalen Ende des Stiels, dieser Abstand B ist bei einem Stiel mit einem Abstand A von 8 mm etwa 6,5 mm, bei einem Stiel mit einem Abstand A von 17 mm dagegen etwa 10,6 mm, innerhalb dieses Bereiches ändert sich der Abstand B proportional zur Zunahme der Größe A.

Entsprechendes gilt für den Abstand C, der zwischen einem Minimalmaß von 14,9 mm und einem maximalen Maß von 19,4 mm schwankt, für den Abstand E, der zwischen einem Minimalabstand von 37,7 mm und einem Maximalabstand von 48,5 mm schwankt, dem Meßabstand X, der zwischen einem Minimalabstand von 18 mm und einem Maximalabstand von 27 mm schwankt, und hinsichtlich der Gesamtlänge L, die zwischen einem Mindestmaß von 142,5 mm und einem Höchstabstand von 183 mm schwankt.

Insbesondere ändert sich der Radius Ra entsprechend den Abmessungen der einzelnen Mitglieder des Satzes, das Mindestmaß dieses Radius liegt bei 2,9 mm für einen Stiel mit einem Abstand A von 8 mm, das Höchstmaß bei 4,7 mm für einen Stiel mit einem Abstand A von 17 mm.

Andere Größen dagegen bleiben unverändert, dies gilt beispielsweise für die öffnungswinkel δ und γ und für den Radius Ri des nach innen gebogenen Abschnittes 17 der Stege 14 und 15 sowie für den Krümmungsradius Rs der Stege 14 und 15.

Auch der Neigungswinkel β zwischen der Längsachse des Steckkonus und der Längsachse des Stieles 2 bleibt unverändert bei etwa 135°.

Damit wird innerhalb des Satzes eine feste Zuordnung der Abmessungen geschaffen, und es hat sich herausgestellt, daß gerade bei dieser festen Zuordnung optimale Bedingungen für das maschinelle Vorbereiten des Markraumes herrschen, so daß in dieser Weise dimensionierte Hüftprothesenschäfte 1 besonders vorteilhaft in Markraumhöhlen implantiert werden können, die in dieser Weise vorbereitet sind.

Die Prothesen können im übrigen in der üblichen Weise aus einem körperverträglichen Metall bestehen, beispielsweise aus Titan oder einer Titanlegierung.

## Patentansprüche

1. Hüftprothesenschaft (1) mit einem eine gerade Längsachse aufweisenden Stiel (2), der an seinem proximalen Ende übergeht in ein verbreitertes Kopfteil (8) mit einem Steckkonus (12) für eine Gelenkkugel, dessen Längsachse gegenüber der Längsachse des Stiels (2) geneigt ist und welches auf gegenüberliegenden Seitenflächen (9, 10) quer abstehende, im wesentlichen in Längsrichtung des Stiels (2) verlaufende Stege (14, 15) trägt, wobei der Stiel (2) im wesentlichen parallel zueinander verlaufende Seitenflächen (3, 4) aufweist, die in im wesentlichen parallel zueinander verlaufende Seitenflächen (9, 10) des Kopfteiles (8) übergehen, wobei die Seitenflächen (3, 4) des Stiels (2) über abgerundete Kanten (7) in eine vordere und in eine hintere Außenfläche (6 bzw. 5) des Stiels (2) übergehen, **dadurch gekennzeichnet, daß** die Seitenflächen (3, 4) des Stiels und die Seitenflächen (9, 10) des Kopfteiles eben sind und der Radius (Ra) der abgerundeten Kanten (7) mindestens 2,9 mm und maximal 5 mm beträgt.

2. Schaft nach Anspruch 1, **dadurch gekennzeichnet, daß** die Stege (14, 15) im Querschnitt je einen nach außen gebogenen Abschnitt (16) und einen daran stetig anschließenden, nach innen gebogenen, stetig in die Seitenflächen (9, 10) des Kopfteils (8) übergehenden Abschnitt (17) aufweisen, wobei der Radius (Ra) des nach außen gebogenen Abschnittes (16) mindestens 2,9 mm beträgt.

3. Schaft nach Anspruch 2, **dadurch gekennzeichnet, daß** die nach außen gebogenen Abschnitte (16) der Stege (14, 15) stetig ineinander übergehen.

4. Schaft nach Anspruch 3, **dadurch gekennzeichnet, daß** der Radius (Ra) der nach außen gebogenen Abschnitte (16) von den Seitenflächen (9, 10) des Kopfteils (8) bis zu ihrem Zusammentreffen an der hinteren Außenfläche (13) zunimmt.

5. Schaft nach einem der Ansprüche 2 bis 4, **dadurch gekennzeichnet, daß** der Radius (Ri) des nach innen gebogenen Abschnitts (17) zwischen 3,0 und 4,0 mm beträgt.

6. Schaft nach einem der voranstehenden Ansprüche, **dadurch gekennzeichnet, daß** die Höhe (D) der Stege (14, 15) relativ zu den Seitenflächen (9, 10) des Kopfteils (8) zwischen 2 und 3 mm liegt.

7. Schaft nach einem der voranstehenden Ansprüche, **dadurch gekennzeichnet, daß** die Stege (14, 15) vom unteren Ende des Kopfteils (8) bis an dessen Oberkante verlaufen und von ihrem unteren Ende, welches parallel zur Längsachse des Stiels (2) verläuft, bis zu ihrem oberen Ende in Richtung auf den Steckkonus (12) gekrümmt sind.

8. Schaft nach Anspruch 7, **dadurch gekennzeichnet, daß** der Krümmungsradius (Rs) der Stege (14, 15) zwischen 100 und 140 mm liegt.

9. Schaft nach einem der voranstehenden Ansprüche, **dadurch gekennzeichnet, daß** die ebenen Seitenflächen (3, 4) des Stieles (2) von dessen distalem Ende ausgehend um einen Winkel (δ) zwischen 1,5° und 2,5° divergieren.

10. Schaft nach einem der voranstehenden Ansprüche, **dadurch gekennzeichnet, daß** die vordere und die hintere Außenfläche (6 bzw. 5) des Stiels (2) von dessen distalem Ende ausgehend um einen Winkel (γ) zwischen 2,5° und 3,5° divergieren.

11. Satz von Hüftprothesenschäften nach einem der voranstehenden Ansprüche, **dadurch gekennzeichnet, daß** der Abstand der vorderen und hinteren Außenflächen (6 bzw. 5) des Stiels (2) zwischen 8 und 17 mm abgestuft ist und daß die Radien (Ra) der abgerundeten Kanten (7) der Seitenflächen (3, 4) des Stieles (2) und die Radien (Ra) der nach außen gebogenen Abschnitte (16) der Stege (14, 15) von einem Mindestradius von 2,9 mm bis zu einem Höchstradius von 4,7 mm proportional zum Abstand der vorderen und hinteren Außenflächen (6 bzw. 5) des Stiels (2) zunehmen.

12. Satz von Hüftprothesenschäften nach Anspruch 12, **dadurch gekennzeichnet, daß** auch der Abstand (B) der Seitenflächen (3, 4) des Stiels (2) zwischen einem Mindestmaß von 6,5 mm und einem maximalen Maß von 10,6 mm proportional zum Abstand der vorderen und der hinteren Außenflächen (6 bzw. 5) des Stiels (2) zunimmt.

13. Satz von Hüftprothesenschäften nach Anspruch 12 oder 13, **dadurch gekennzeichnet, daß** auch die Gesamtlänge (L) des Hüftprothesenschafts (1) zwischen einem Mindestmaß von 140 mm und einem Höchstmaß von 183 mm proportional zum Abstand (A) der vorderen und hinteren Außenflächen (6 bzw. 5) des Stieles (2) zunimmt.

## Claims

1. A shaft (1) for a hip prosthesis, comprising a stem (2) having a straight longitudinal axis and merging at its proximal end into a broadened head part (8) with an insertion cone (12) for a ball of a joint, the longitudinal axis of the head part (8) being inclined relative to the longitudinal axis of the stem (2), and the head part (8) carrying transversely projecting ribs (14, 15) extending substantially in the longitudinal direction of the stem (2) on opposing lateral surfaces (9, 10), wherein the stem (2) has lateral surfaces (3, 4) extending substantially parallel to one another and merging into lateral surfaces (9, 10) of the head part (8) extending substantially parallel to one another, wherein the lateral surfaces (3, 4) of the stem (2) merge via rounded edges (7) into a front and a rear outer surface (6, 5 respectively) of the stem (2), **characterised in that** the lateral surfaces (3, 4) of the stem and the lateral surfaces (9, 10) of the head part are flat and the radius (Ra) of the rounded edges (7) is at least 2.9 mm and at most 5 mm.

2. A shaft according to claim 1, **characterised in that** the ribs (14, 15) in cross-section each have an outwardly curved portion (16) and a continuously adjacent, inwardly curved portion (17) merging continuously into the lateral surfaces (9, 10) of the head part (8), the radius (Ra) of the outwardly curved portion (16) being at least 2.9 mm.

3. A shaft according to claim 2, **characterised in that** the outwardly curved portions (16) of the ribs (14, 15) merge continuously into one another.

4. A shaft according to claim 3, **characterised in that** the radius (Ra) of the outwardly curved portions (16) increases from the lateral surfaces (9, 10) of the head part (8) to the point at which they meet at the rear outer surface (13).

5. A shaft according to any one of claims 2 to 4, **characterised in that** the radius (Ri) of the inwardly curved portion (17) is between 3.0 mm and 4.0 mm.

6. A shaft according to any one of the preceding claims, **characterised in that** the height (D) of the ribs (14, 15) in relation to the lateral surfaces (9, 10) of the head part (8) is between 2 mm and 3 mm.

7. A shaft according to any one of the preceding claims, **characterised in that** the ribs (14, 15) extend from the lower end of the head part (8) to its upper edge and are curved from their lower end, which extends parallel to the longitudinal axis of the stem (2), to their upper end in the direction of the insertion cone (12).

8. A shaft according to claim 7, **characterised in that** the radius of curvature (Rs) of the ribs (14, 15) lies between 100 mm and 140 mm.

9. A shaft according to any one of the preceding claims, **characterised in that** the flat lateral surfaces (3, 4) of the stem (2) diverge from its distal end through an angle (δ) of between 1.5° and 2.5°.

10. A shaft according to any one of the preceding claims, **characterised in that** the front and the rear outer surfaces (6, 5) of the stem (2) diverge from its distal end through an angle (γ) of between 2.5° and 3.5°.

11. A set of shafts for hip prostheses according to any one of the preceding claims, **characterised in that** the distance between the front and rear outer surfaces (6, 5) of the stem (2) varies between 8 mm and 17 mm and **in that** the radii (Ra) of the rounded edges (7) of the lateral surfaces (3, 4) of the stem (2) and the radii (Ra) of the outwardly curved portions (16) of the ribs (14, 15) increase from a minimum radius of 2.9 mm to a maximum radius of 4.7 mm in proportion to the distance between the front and rear outer surfaces (6, 5) of the stem (2).

12. A set of shafts for hip prostheses according to claim 12 [sic], **characterised in that** the distance (B) between the lateral surfaces (3, 4) of the stem (2) also increases between a minimum measurement of 6.5 mm and a maximum measurement of 10.6 mm in proportion to the distance between the front and the rear outer surfaces (6, 5) of the stem (2).

13. A set of shafts for hip prostheses according to claim 12 or 13 [sic], **characterised in that** the overall length (L) of the hip prosthesis shaft (1) also increases between a minimum measurement of 140 mm and a maximum measurement of 183 mm in proportion to the distance (A) between the front and rear outer surfaces (6, 5) of the stem (2).

## Revendications

1. Tige de prothèse de hanche (1) comportant un fût (2) présentant un axe longitudinal droit, qui se transforme à son extrémité proximale en une partie de tête (8) élargie avec un cône d'enfichage (12) pour une bille d'articulation, dont l'axe longitudinal est incliné par rapport à l'axe longitudinal du fût (2) et qui porte sur des surfaces latérales (9, 10) opposées des ailes (14, 15) faisant saillie transversalement et s'étendant sensiblement en direction longitudinale du fût (2), le fût (2) présentant des surfaces latérales (3, 4) sensiblement parallèles l'une à l'autre, qui se transforment en des surfaces latérales (9, 10) sensiblement parallèles l'une à l'autre de la partie de tête (8), les surfaces latérales (3, 4) du fût (2) se transformant via des arêtes (7) arrondies en une surface extérieure antérieure (6) et en une surface extérieure postérieure (5) du fût (2), **caractérisée en ce que** les surfaces latérales (3, 4) du fût et les surfaces latérales (9, 10) de la partie de tête sont planes, et **en ce que** le rayon (Ra) des arêtes (7) arrondies est d'au moins 2,9 mm et au maximum de 5 mm.

2. Tige selon la revendication 1, **caractérisée en ce que** les ailes (14, 15) présentent chacune en section transversale un tronçon (16) recourbé vers l'extérieur et un tronçon (17) qui se raccorde de façon continue à celui-ci, recourbé vers l'intérieur et se transformant de façon continue pour former les surfaces latérales (9, 10) de la partie de tête (8), le rayon (Ra) du tronçon (16) recourbé vers l'extérieur étant d'au moins 2,9 mm.

3. Tige selon la revendication 2, **caractérisée en ce que** les tronçons (16) recourbés vers l'extérieur des ailes (14, 15) se transforment de façon continue l'un dans l'autre.

4. Tige selon la revendication 3, **caractérisée en ce que** le rayon (Ra) des tronçons (16) recourbés vers l'extérieur augmente depuis les surfaces latérales (9, 10) de la partie de tête (8) jusqu'à leur rencontre avec la surface extérieure postérieure (13).

5. Tige selon l'une des revendications 2 à 4, **caractérisée en ce que** le rayon (Ri) du tronçon (17) recourbé vers l'intérieur est entre 3,0 et 4,0 mm.

6. Tige selon l'une des revendications précédentes, **caractérisée en ce que** la hauteur (D) des ailes (14, 15) par rapport aux surfaces latérales (9, 10) de la partie de tête (8) est entre 2 et 3 mm.

7. Tige selon l'une des revendications précédentes, **caractérisée en ce que** les ailes (14, 15) s'étendent depuis l'extrémité inférieure de la partie de tête (8) jusqu'à son arête supérieure et sont recourbées depuis leur extrémité inférieure, qui s'étend parallèlement à l'axe longitudinal du fût (2), jusqu'à leur extrémité supérieure en direction du cône d'enfichage (12).

8. Tige selon la revendication 7, **caractérisée en ce que** le rayon de courbure (Rs) des ailes (14, 15) est entre 100 et 140 mm.

9. Tige selon l'une des revendications précédentes, **caractérisée en ce que** les surfaces latérales planes (3, 4) du fût (2) divergent d'un angle (δ) entre 1,5° et 2,5° à partir de son extrémité distale.

10. Tige selon l'une des revendications précédentes, **caractérisée en ce que** la surface extérieure antérieure (6) et la surface extérieure postérieure (5) du fût (2) divergent d'un angle (γ) entre 2,5° et 3,5° à partir de son extrémité distale.

11. Jeu de tiges de prothèses de hanche selon l'une des revendications précédentes, **caractérisé en ce que** la distance entre la surface extérieure antérieure (6) et la surface extérieure postérieure (5) du fût (2) est étagée entre 8 et 17 mm, et **en ce que** les rayons (Ra) des arêtes arrondies (7) des surfaces latérales (3, 4) du fût (2) et les rayons (Ra) des tronçons (16) recourbés vers l'extérieur, des ailes (14, 15), augmentent depuis un rayon minimum de 2,9 mm jusqu'à un rayon maximum de 4,7 mm proportionnellement à la distance de la surface extérieure antérieure (6) et de la surface extérieure postérieure (5) du fût (2).

12. Jeu de tiges de prothèses de hanche selon la revendication 11, **caractérisé en ce que** la distance (B) entre les surfaces latérales (3, 4) du fût (2) augmente entre une dimension minimum de 6,5 mm et une dimension maximum de 10,6 mm proportionnellement à la distance entre la surface extérieure antérieure (6) et la surface extérieure postérieure (5) du fût (2).

13. Jeu de tiges de prothèses de hanche selon l'une ou l'autre des revendications 11 et 12, **caractérisé en ce que** la longueur totale (L) de la tige (1) de prothèse de hanche augmente entre une dimension minimum de 140 mm et une dimension maximum de 183 mm proportionnellement à la distance (A) entre la surface extérieure antérieure (6) et la surface extérieure postérieure (5) du fût (2).
